# EUROPEAN PATENT SPECIFICATION

(11) **EP 2 129 433 B1**
(45) Date of publication and mention of the grant of the patent: **16.10.2013**
(21) Application number: 07835237.4
(22) Date of filing: 27.11.2007
(51) Int. Cl.: A61N 1/37, A61B 5/02, A61B 5/08, A61N 1/02, A61B 5/145, A61B 5/1459, A61N 1/05, A61N 1/365

(54) **IMPLANTABLE MEDICAL DEVICE COMPRISING AN OXYGEN SENSOR**
IMPLANTIERBARES MEDIZINPRODUKT MIT EINEM SAUERSTOFFSENSOR
DISPOSITIF MÉDICAL IMPLANTABLE COMPRENANT UN CAPTEUR D'OXYGÈNE

(30) Priority: 23.03.2007 WO PCT/SE2007/000289
(43) Date of publication of application: 09.12.2009
(73) Proprietor: St. Jude Medical AB, 175 84 Järfälla (SE)
(72) Inventor: HEDBERG, Sven-Erik, S-196 32 Kungsängen (SE); BLOMQVIST, Andreas, SE - 187 76 Täby (SE); JÄRVERUD, Karin, S-169 71 Solna (SE); LJUNGSTRÖM, Karin, S-165 70 Hässelby (SE); LINDKVIST, Leif, S-179 62 Stenhamra (SE); LÖNN, Urban, SE-756 45 Uppsala (SE); HOLMSTRÖM, Nils, S-175 49 Järfälla (SE)
(74) Representative: Sjölander, Henrik
(86) International application number: PCT/SE2007/001044
(87) International publication number: WO 2008/118047

(56) References cited:
- EP-A2- 0 252 578
- EP-A2- 1 102 198
- EP-A2- 1 151 718
- WO-A1-00/13003
- WO-A1-00/25664
- WO-A1-00/38575
- WO-A1-2004/091696
- WO-A1-2005/110532
- US-A- 4 860 751
- US-A- 5 076 271
- US-A- 5 275 171
- US-A- 5 431 172
- US-A- 5 498 549
- US-A- 5 498 549
- US-A1- 2004 082 841
- US-A1- 2005 065 556
- US-A1- 2005 216 064
- US-A1- 2007 027 496
- US-B1- 6 198 952

## Description

### Field of the invention

The present invention relates to a device according to the preamble of the independent claim.

### Background of the invention

Insufficiency of the lungs, like in Chronic Obstructive Pulmonary Disease (COPD), pulmonary edema due to Heart Failure, asthma, chronic bronchitis, emphysema and interstitial lung diseases have high prevalence adding extensive costs to the health care system and suffering for the patients. Therefore, it is a general fact that enhanced management of these patients would be useful.

In the following a number of publications are briefly discussed providing relevant background information in the field of diagnostic methods and apparatuses for detecting insufficiencies of the lungs.

US 2003/0149367 relates to a system and method for evaluating risk of mortality due to congestive heart failure using physiological sensors. The method includes that arterial oxygen saturation is measured. Electrodes may be place in the left atrium for atrial pacing. It is mentioned that a combined pacemaker lead and physiological sensor can be used.

EP-1102198 relates to an automated collection and analysis patient care system and method for diagnosing and monitoring respiratory insufficiency and outcomes thereof. The system includes an implantable device for measuring the arterial oxygen saturation.

EP-1151718 relates to an apparatus for monitoring heart failure via respiratory patterns. An implantable device is adapted to measure the arterial oxygen saturation in order to detect changes in the breathing pattern.

US-7,010,337 discloses method and apparatus for monitoring blood condition and cardiopulmonary function, where sensors located on a sensor carrier are placed adjacent one or more of a surgical patient's major thoracic blood-containing structures such as the aorta or pulmonary artery, and characteristics of the blood in the blood-containing structures are determined non-invasively by measuring transmission or reflection of light or other types of energy by the blood.

US-6,198,952 discloses an implantable sensor assembly for use with an implantable medical device according to the first part of claim 1. The sensor assembly comprises an oxygen sensor in the form of an elongated housing with a light emitter, a light detector and two light transmissive lenses separated by and spaced apart so that light from the light emitter emitted through a first lens is not transmitted directly into the second lens.

US 2004/0082841 discloses a carrier with an oxygen sensor at its distal and designed to be placed adjacent and around a blood vessel in order to optically detect characteristics of the blood flowing through the blood vessel.

The inventors have realized that further improvements of today's technology are needed in order to meet the increasing demands of devices that enable accurate and reliable detection of lung insufficiencies.

Therefore, the object of the present invention is to achieve a device that has improved capabilities regarding detection and monitoring of a patient's lung function, which may be related to Chronic Obstructive Pulmonary Disease (COPD), pulmonary oedema due to Heart Failure, asthma, chronic bronchitis, emphysema and interstitial lung diseases.

### Summary of the invention

The above-mentioned object is achieved by the present invention according to the independent claim.

Preferred embodiments are set forth in the dependent claims.

According to the present invention the degree of oxygen saturation of the blood oxygenized by the lungs, is monitored by the means of an optical blood oxygen saturation sensor.

In particular the oxygen saturation measurement is performed when specified measurement criteria is fulfilled.

According to the invention the sensor is to be placed at the left atrium in the septum wall between the right and left atria. The left atrium, directly receives the oxygenized blood from the lungs.

According to another embodiment the oxygen sensor is mechanically integrated or co-assembled with a pressure sensor.

The sensor is connected to an implantable medical device (IMD). The device is provided with measuring means enabled to measure the blood oxygen saturation by the oxygen sensor at suitable points in time. At a time, when the patient carrying this IMD, is at rest or asleep, the interference from other sources, that might disturb the sensor measurements, preferably is at a minimum. This is often the best choice of time to perform these measurements. The blood leaving the lungs has normally a high SaO₂ value even during high patient workload.

According to an embodiment of the present invention, the degree of oxygen saturation of the blood oxygenized by the lungs is monitored by means of photoluminescence sensor utilizing photoluminescent molecules emitting light as a response to the concentration of oxygen in blood.

In embodiments of the invention, the oxygen sensor is a pO₂ sensor, for example, a sensor as described in US 5,431,172 sensitive to the levels of oxygen in arterial blood. It has been shown that the SvO₂ and SaO₂ values are correlated with the partial pressure of oxygen, pO₂, in blood and thus SaO₂ values may easily be calculated from measured pO₂ values, and vice versa.

In case a lung problem evolves, which causes inadequate oxygenation, this will be measured by the sensor. SaO₂ data will be stored and time marked in the IMD and analyzed. The stored data shall be used for diagnostic purposes and by the algorithm which will detect when the SaO₂ values are below a certain limit for more than a certain period of time and at conditions which are recognized to give reliable sensor data. Normal patient activity will be recognized by data from an auxiliary sensor, such as an activity sensor. The SaO₂ data should normally only be accepted when the activity sensor data indicate a patient at rest. In order to get reliable sensor data they shall be sampled at several times during this period of inactivity and the data shall be at about the same amplitude.

Disturbing influences, such as flying at a high altitude, may result in wrong interpretation of the status of the patient. Other auxiliary sensors, such as accelerometers and posture sensors, are then useful for being able to distinguish flying at high altitude from having impaired oxygenation in the lungs, e.g. by not performing blood oxygen saturation measurements if auxiliary sensors indicate that the patient is flying at a high altitude.

The present invention may also be used for detecting when a patient no longer may saturate the blood during exercise. This may be used for diagnostic purposes to show the physician at which work load this desaturation starts.

The usage of a SaO₂ sensor in the left atrium may also be used together with saturation sensors in other parts of the heart or body of the patient. In many cases those algorithms using blood saturation sensors anticipate full saturation of the blood leaving the lungs. An example of that would be in a pacemaker provided with a rate responsive function based on the oxygen saturation in the right ventricle.

The SaO₂ value may also be used for detecting impaired oxygenation in the lungs caused by pulmonary edema. The SaO₂ sensor may not differentiate pulmonary edema from other lung diseases, but using impedance measurements internally over parts of thorax may indicate that such a condition is at hand.

SaO₂-values may be transferred to e.g. databases and physicians using conventional telemetry communication and further on via internet on regular or planned occasions as well as when SaO₂ alarm limits have been passed.

### Short description of the appended drawings

Figure 1 is a schematic block diagram illustrating the present invention.
Figure 2 is a schematic block diagram illustrating one embodiment of the present invention.
Figure 3 is a schematic block diagram illustrating another embodiment of the present invention.
Figure 4 is a schematic illustration of an electrode lead according to the present invention, where the oxygen sensor is arranged; a sectional side view, and a view from the distal side of the lead (to the right in the figure).
Figure 5 is a schematic illustration of an electrode lead according to one embodiment of the present invention, where the oxygen sensor is arranged; a sectional side view, and a view from the distal side of the lead (to the right in the figure).
Figure 6 is a schematic illustration of an implantable medical device according to the invention provided with two electrode leads.
Figure 7 is a schematic illustration of the p O₂ sensor according to one embodiment of the present invention.
Figure 8 is a schematic illustration of the p O₂ sensor according to one embodiment of the present invention.

### Detailed description of preferred embodiments of the invention

The oxygen saturation of blood is generally defined as the percentage of the hemoglobin binding sites that are occupied with an oxygen molecule. An SaO₂ value in the range of 97-100% is considered normal, and an SaO₂ value in the range of 92-97% indicates interstitial edema and should trigger an alarm to avoid pulmonary edema and hospitalization (this is further discussed below). An SaO₂ value below 92% indicates pulmonary edema and the patient should immediately come to hospital.

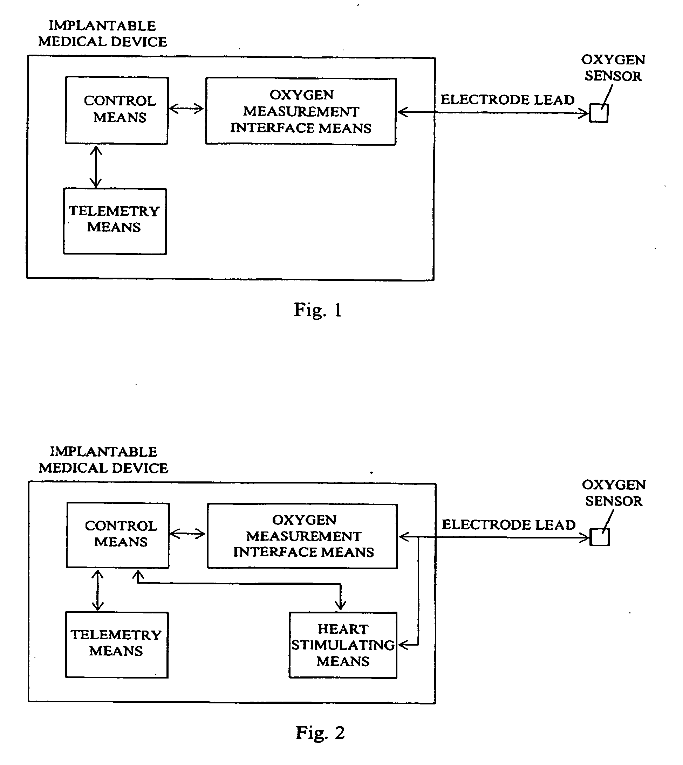

Figure 1 is a schematic block diagram illustrating the present invention. The implantable medical device according to the present invention comprises an optical oxygen sensor adapted to measure the level of oxygen in oxygenized blood, and to generate an oxygen measurement signal in dependence of the level of oxygen. The oxygen measurement signal is applied to an oxygen measurement interface means that filters and amplifies the signal and then applies the signal to a control means. The oxygen sensor is arranged to perform measurements inside the heart, of blood entering the left atrium of a patient's heart and is preferably arranged at, or close to, a distal end of an electrode lead connected to the medical device. The obtained oxygen measurement signal is compared, by the control means, to a predetermined threshold level and an indication signal, indicative of the lung functionality of the patient, is generated in dependence of said comparison. Both the obtained oxygen measurement signal as well as the indication signal are stored in a storage means (not shown) arranged in the control means. The stored signals are time-stamped.

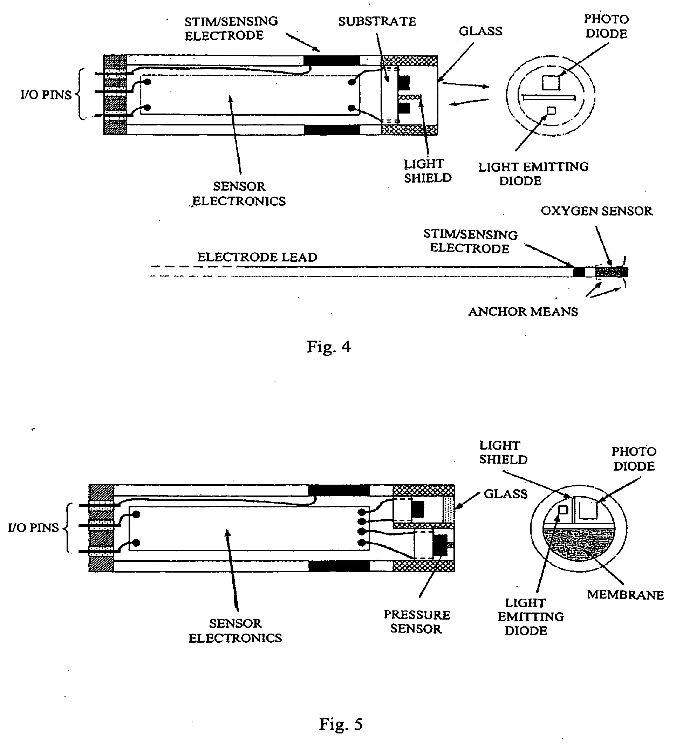

As illustrated in figure 4 the optical sensor preferably comprises a photo-diode and a light emitting diode arranged at the distal tip of the electrode lead and facing the distal direction of the lead. The photo diode and the light emitting diode is mounted at a substrate and separated by a light shield. The sensor is protected by a glass cover that is transparent for the relevant wavelengths emitted by the diode. Sensor electronics are also included in connection with the optical sensor. Input/output pins are arranged to connect the sensor electronics to electrical conductors (not shown) of the electrode lead and that in turn are connected to the oxygen measurement interface means.

According to one embodiment of the present invention the implantable medical device is a heart stimulator which is illustrated in figure 2. The medical device as described with references to figure 1 then includes a heart stimulating means and the electrode lead includes one or many sensing and stimulating electrodes. One stimulating/sensing electrode is shown in figure 4. Naturally, more than one electrode lead may be connected to the medical device adapted to be arranged both in the left and right chambers of the heart. In this embodiment the oxygen measurement signal may be used to control the stimulation rate of the heart stimulator.

The electrode lead provided with the optical sensor is adapted to be placed in the left atrium of the heart and in particular to perform measurements in the left atrium from a position reached from the right atrium via the septal wall between the right and left atrium of the heart. To firmly anchor and attach the distal tip within the septal wall the distal end of the electrode lead is provided with anchor means. The anchor means is schematically illustrated in the lower part of figure 4 where the distal end of an electrode lead is shown. The anchor means preferably comprises at least two hatches separately arranged along the distal tip of the electrode lead.

As briefly mentioned above an indication signal is generated if the comparison indicates impaired lung functionality of the patient, e.g. to detect when a patient no longer may saturate the blood during exercise. In particular the indication signal is generated if the measurement signal is below a predetermined oxygen level threshold longer than a preset time period. A predetermined oxygen level threshold is 95% SaO₂.

The threshold may naturally be set to any other value, depending on the purpose of the measurement. As stated above, normal values of SaO₂ are typically 97-100% and if the value is below 92%, then the patient has severe problems.

In embodiments of the invention, the oxygen sensor is a pO₂ sensor, for example, a sensor as described in US 5,431,172 sensitive to the levels of oxygen in arterial blood. It has been shown that the SvO₂ and SaO₂ values are correlated with the partial pressure of oxygen, pO₂, in blood and thus SaO₂ values may easily be calculated from measured pO₂ values, and vice versa.

In one embodiment of the present invention, the implantable medical device comprises an oxygen sensor, wherein the oxygen sensor is adapted to perform measurements inside the heart, of blood entering the left atrium of a patient's heart, to obtain a partial pressure of oxygen, pO₂, dissolved in the blood, an SaO₂ signal corresponding to the obtained pO₂ signal is calculated, and the calculated SaO₂ signal is compared to a predetermined threshold level and an indication signal is generated in dependence of said comparison, wherein said indication signal is indicative of the lung functionality of the patient.

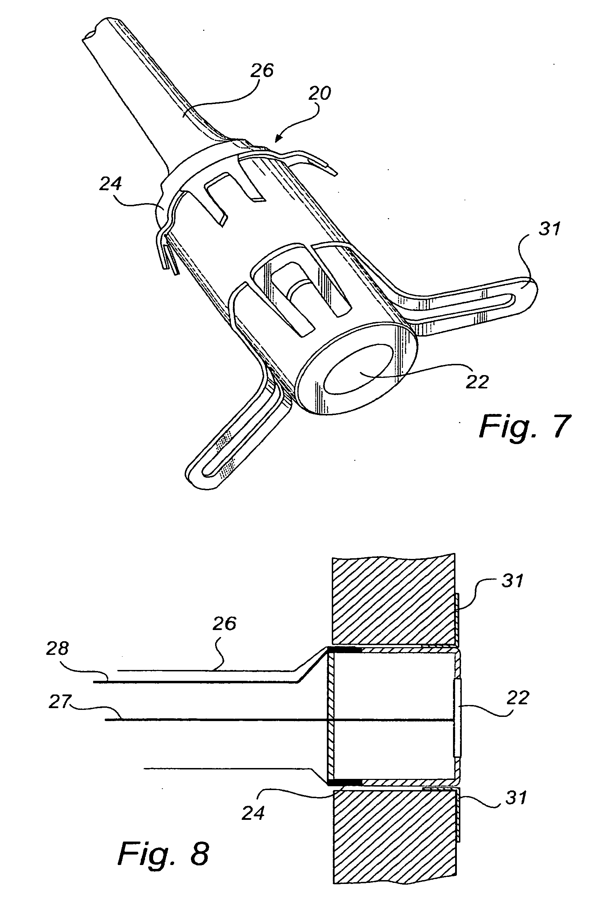

With reference now to Figs. 7 and 8, one particular embodiment of the oxygen sensor will be discussed. The oxygen sensor 20 is arranged to penetrate septum 23 to sense the oxygen concentration of the blood in left atrium 30. The sensor comprises a working electrode 22 on which an outer surface is exposed to the blood in the left atrium 30, i.e. the sensor is located such that the outer surface of the working electrode 22 is in direct contact with the arterial blood 30. The reduction of oxygen takes place on the outer surface of the working electrode 22. According to one embodiment, the outer surface of the working electrode 22 has a coating of gold. It has been shown that the tissue overgrowth on negatively charged gold surfaces in venous blood is limited and stable over time (Holmström et.al. in Colloids and Sufaces B: Biointerfaces 11 (1998) 265-271).

Further, the sensor comprises a counter electrode (not shown) adapted to emit electrical current to the system and a reference electrode 24 adapted to sense an electrical potential in the blood or tissue to ensure a proper measurement potential. The sensor is connected to the implantable medical device via an implantable medical lead 26 including connections 27, 28 to the working electrode 22 and the reference electrode, respectively.

According to an embodiment, the sensor 20 is implanted transvenously and, as mentioned above, penetrates the septum 23 to place the outer surface of the working electrode 22 in contact with the blood in left atrium 30. The sensor is fixated to septum 30 by means of fixation means 31, for example, fixation plates or fixation elements.

Another embodiment of the present invention, a sensor for measuring the concentration of oxygen in blood by means of photoluminescence is arranged in the device for monitoring progression of heart failure in a human heart according to the present invention. Such a sensor is described in PCT/SE2007/000410 ("IMPLANTABLE DEVICES AND METHOD FOR DETERMINING A CONCENTRATION OF A SUBSTANCE AND/OR MOLECULE IN BLOOD OR TISSUE OF A PATIENT").

The sensor comprises a carrier in which photoluminescent molecules are embedded. The carrier is partially in contact with blood of a patient comprising oxygen for which the concentration shall be determined. The sensor further comprises a light source and a photo-detector which are optically connected to the carrier such that the light emitted from the light source can excite the photoluminescent molecules and such that the light emitted from the photoluminescent molecules in response to this excitation can be detected by the photo-detector. The carrier is selectively permeable to the oxygen molecules for which the concentration has to be determined such that oxygen molecules can diffuse from the region of the patient into the carrier. The photoluminescent molecules are selected to react with oxygen in the blood in such a manner that the characteristics of the light emitted from the photoluminescent molecules is altered because of the reaction. Depending on the concentration of oxygen molecules in the blood of the patient, the characteristics of the light emitted from the photoluminescent molecules will then be altered to different degrees. Thus, a determination of the concentration of oxygen in the blood of the patient is enabled.

In an embodiment, the light source and the photo-detector are directly arranged at a side of the carrier or in the carrier. In another embodiment, the light source and the photo-detector are optically connected to the photoluminescent molecules comprised in the carrier by means of optical fibers. The optical fibers may be arranged between the light source and the photoluminescent molecules and/or between the photo-detector and the photoluminescent molecules, which means that the light source and the photo-detector can be arranged at a certain distance from the analyzed region. The optical fibers are used to guide light from the light source and to guide light to the photo-detector, respectively.

In an embodiment, the light source and the photo-detector are fabricated on a same substrate using e.g. standard semiconductor technology. The carrier is then made of a material, such as a silicon adhesive, like e.g. the commercially available Rehau RAU-SIK SI-1511, containing the photoluminescent molecules and spread over the substrate. The substrate can then be used as a support for the light source, the photo-detector and the carrier. Such a substrate would also enable easy connection between the light source, the photo-detector and other components used to analyze the signal output of the photo-detector. The thickness of the adhesive film containing the photoluminescent molecules would typically be comprised between 0.03 and 3 mm.

In yet a further embodiment, the light source and the photo-detector can be fabricated on two separate substrates and joined together by means of the carrier, e.g. the silicon adhesive mentioned above, containing the photoluminescent molecules.

In an embodiment, a filter may be arranged in front of the photo-detector to select the range of wavelength that shall be transmitted to the photo-detector. In particular, the filter is used to eliminate the part of the excitation light emitted by the light source that is reflected by the photoluminescent molecules or by the carrier or carrier matrix.

In further embodiments, the carrier can be made as a material comprised in the group of silicone rubber (also known as polydimethylsiloxane), organosubstitute silicones such as poly(R-R'-siloxane) wherein R and R' are one of the following {methyl, ethyl, propyl, butyl, "alkyl", "aryl", phenyl and "vinyl"} and not necessarily equal to each other, polyurethane, poly(1-trimethylsily-1-propyne), polystyrene, poly(methylmethacrylate), poly(vinyl chloride), poly(isobutylmethacrylate), poly(2,2,2-trifluoroethylmethacrylate), ethylcellulose, cellulose acetobutyrate, cellulose acetate, gas-permeable polytetrafluoroethylene and thermoplastic polyurethanes and copolymers, in which material the photoluminescent molecules are embedded. Further, these materials, in particular silicone rubber, thermoplastic polyurethanes and copolymers, gas-permeable polytetrafluoroethylene, are well adapted in the present invention since they are implantable biomaterials. In a particular embodiment, silicone rubber is well adapted if the sensor 1 is used to determine the concentration of oxygen in the region 10 since its permeability to oxygen is about ten times higher than in most polymeric materials, namely 6.95×10¹¹ cm⁻²s⁻¹Pa⁻¹, which corresponds to a diffusion coefficient of about 1.45×10⁻⁵ cm²s⁻¹.

The light source may be a solid state light source, preferentially a light emitting diode, which is advantageous since light emitting diodes are small and can therefore easily be incorporated in the sensor. Light emitting diodes are advantageous since the intensities, wavelengths, and time responses are controllable. Light emitting diodes can emit at various ranges, which generally extend from 390 nm to 1650 nm although not any wavelength of this range may be achieved.

The sensitivity of the photo-detector of the implantable sensor depends on the wavelength range at which the photoluminescent molecules emit. Generally, the range of wavelength at which photoluminescent molecules emit extend from 350 to 1800 nm, in particular in the range of 350-800 nm. Thus, the photo-detector shall be sensitive in this range of wavelength. As the wavelength at which selected photoluminescent molecules emit is known, the sensitivity of the photo-detector can be selected accordingly. As an example, oxyphors emit light at about 800 nm, which corresponds to near infrared. In this case, the photo-detector may be a commercially available planar InGaAs photodiode, which is sensitive to red and infrared light, i.e. in the spectral range of 800-1800 nm. Such a detector has a response time of up to 120 MHz, which is sufficiently rapid for measuring photoluminescent lifetimes and sufficiently quantitative for steady state measurements of intensities as well. The measurements of lifetimes and intensities will be described in more details later. In particular, the photo-detector may be one of the group comprised of a pn photodiode, a pin photodiode, a Schottky photodiode, an avalanche photodiode, a silicon photodiode, a planar InGaAs photodiode, a SiGe-based optoelectronic circuit and a InGaN/GaN multiple quantum well pn junction.

The photoluminescent molecules may be fluorescent molecules or phosphorescent molecules. Although it would be preferable to use fluorescent molecules as they are generally more stable over time, it is preferable to use phosphorescent molecules as phosphorescence leads to longer timescale, which then facilitates the design of the electronics in the sensor.

Since the sensor is designed to determine the concentration of oxygen in blood, it is preferable that the photoluminescent molecules are not sensitive to other gases than oxygen such as carbon monooxide which can also be found in blood. The photoluminescent molecules shall also be easily bounded to the material of the carrier, i.e. they should be compatible with the characteristics of the carrier.

Photoluminescent molecules that may be used to determined the concentration of oxygen are molecules comprised within the group of pyrene, pyrene derivatives (vinylpirene, methoxypyrene), a polyaromatic carrier, an ionic probe (ruthenium trisbypyridine) and Pd-tetra (4-carboxyphenyl) benzoporphin (Oxyphor). Additional examples are naphthalene derivatives (e.g. 2-dimethylamino-6-lauroylnaphthalene); polypyridyl complexes of transition metals, particularly Ruthenium, Osmium, or Rhenium containing fluorescent molecules {e.g. Ru(bipy)(3)(2+) (tris(2,2'-bipyridyl)ruthenium(II) chloride hexahydrate) and Ru(phen)(3)(2+) (tris(1,10-phenanthroline)ruthenium(II) chloride hydrate)}; fullerenes (C60 and C70); decacyclene; perylene and perylene derivatives (e.g. - perylene dibutylate); and metalloporphines especially Pt(II), Zn(II) and Pd(II) variants.

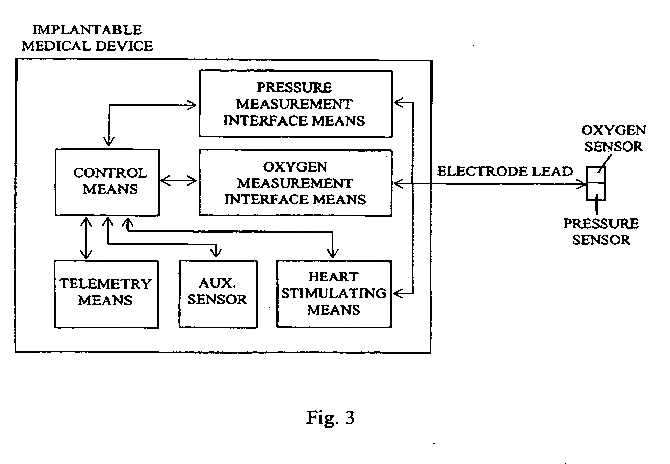

Figure 3 illustrates another preferred embodiment of the present invention where the medical device, in addition to the oxygen sensor, further comprises a pressure sensor arranged in connection with the oxygen sensor. In the figure is also illustrated a heart stimulating means and an auxiliary sensor (to be discussed below). Both the heart stimulating means and the auxiliary sensor are optional.

By including a pressure sensor in connection with the oxygen sensor as illustrated in figure 5 a very compact and versatile electrode lead is provided. As illustrated the pressure sensor is arranged at, or close to, the distal end of an electrode lead connected to the medical device.

In combination with a heart stimulating means, both the oxygen saturation signal and a pressure signal may be used to control the stimulation rate.

The medical device comprises a pressure measurement interface means connected to the pressure sensor.

Different types of pressure sensors may be used. In figure 5 the pressure sensor is a strain gauge pressure sensor attached to the inner side of a membrane. According to another embodiment the pressure sensor is based upon MEMS technology. In short, Micro-Electro-Mechanical Systems (MEMS) is the integration of mechanical elements, sensors, actuators, and electronics on a common silicon substrate through microfabrication technology. While the electronics are fabricated using integrated circuit (IC) process sequences (e.g., CMOS, Bipolar, or BICMOS processes), the micromechanical components are fabricated using compatible "micromachining" processes that selectively etch away parts of the silicon wafer or add new structural layers to form the mechanical and electromechanical devices.

As in the embodiment illustrated in figure 4 the embodiment in figure 5 includes input/output pins connected to electrical conductors of the electrode lead. For the other features of figure 5, not explicitly described here, it is referred to the description of figure 4.

As briefly mentioned above a further embodiment of the medical device comprises an auxiliary sensor adapted to generate an auxiliary measurement signal in dependence of a measurement influencing activity. The blood oxygen measurements are then performed when the auxiliary measurement signal fulfils specified measurement criteria. The reason to include an auxiliary sensor is to ensure that the oxygen measurements are performed with minimal disturbance or interference from other sources, e.g. when the patient is at rest or asleep.

The auxiliary sensor may be an activity sensor, a patient position sensor, or an impedance sensor. The impedance sensor preferably measures the impedance between the electrode lead and the conductive encapsulation of the implantable medical device.

The specified measurement criteria includes that the auxiliary measurement signal is below a specified level, or as an alternative, that the auxiliary measurement signal is above a specified level.

The following is an exemplary description of the function of the implantable medical device provided with an activity sensor, also functioning as a position sensor, and an impedance sensor as two auxiliary sensors.

Limit sensor values are initially set for activity and position, and limits are also set for persistence of activity and rest. The control means then starts to receive data from the position, activity and impedance sensors. Dedicated counters in the control means are increased as a result if detected rest and supine position, respectively, and when these counters have passed its respective limit, status flags are set to "1". If both flags are "1" the SaO₂ measurement starts and the measurement is performed as long as the status flags indicate that the measurement conditions are acceptable.

When the oxygen measurement is performed during a predetermined time interval, e.g. 10-30 minutes, an average value is determined and compare to a threshold as discussed above. If the average value is below the threshold an impedance measurement between electrodes in relevant position in thorax may be performed in order to confirm possible pulmonary edema. An alarm may then be generated via the telemetry communication means to an external device to alert the physician.

As an alternative, the measured SaO₂ values are stored in a storage means in the control means to be further analyzed at a later follow-up procedure.

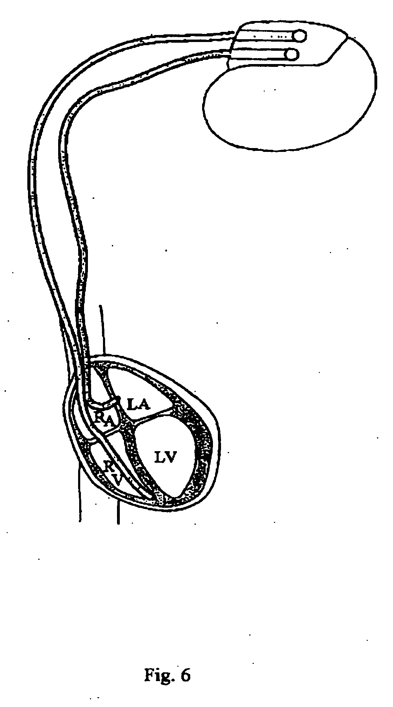

Figure 6 illustrates an exemplary implantable medical device where the present invention is applicable. The illustrated medical device is a two-chamber heart stimulator provided with one electrode lead arranged in the right ventricle (RV) and another electrode lead, including an oxygen senor at the distal tip, arranged in the right atrium (RA), and in particular in the septal wall between the right and left atria and arranged to perform oxygen measurements in the left atrium (LV).

Even though the present invention in particular relates to embodiments showing SaO₂ measured parameters, other oxygen related parameters may be used such as pO₂ as disclosed in US-6,236,873. Measurement electrodes, adapted to perform pO₂ measurements, are then included at the distal part of the electrode lead, and the control means then includes specific hardware and/or software to handle the measurements.

The present invention is not limited to the above-described preferred embodiments. Various, modifications may be used. Therefore, the above embodiments should not be taken as limiting the scope of the invention which is defined by the appending claims.

## Claims

1. Implantable medical device comprising:
an interface means connected to an electrode lead comprising an optical oxygen sensor adapted to measure a level of oxygen in oxygenized blood and generate an oxygen measurement signal in dependence of said level of oxygen, said implantable medical device further comprising a control means connected to said interface means and adapted to compare said oxygen measurement signal to a predetermined threshold level and generate, in dependence of said comparison, an indication signal indicative of lung functionality of said patient,
said oxygen sensor being arranged at a distal end of said electrode lead,
**characterized in that:**
said electrode lead is a right atrial lead comprising anchor means to attach the distal tip of said electrode lead within a septal wall between a right atrium and a left atrium of a patient's heart and, said sensor being arranged at the distal tip and facing the distal direction of said electrode lead, so as to allow said oxygen sensor to perform measurements inside said heart of blood entering said left atrium.

2. Medical device according to claim 1, wherein said optical sensor comprises a photo-diode and a light emitting diode arranged at a distal tip of said electrode lead and facing a distal direction of said electrode lead.

3. Medical device according to claim 1, wherein said oxygen sensor comprises:
at least one light source adapted to, during measurement sessions, emit light at least at a first wavelength;
at least one photo-detector; and
at least one type of photoluminescent molecules embedded in a carrier selectively permeable to oxygen, having a part that is in contact with arterial blood of said patient and being optically connected to said at least one light source and said at least one photo-detector, wherein said photoluminescent molecules emit light in response to excitation by said light emitted from said at least one light source and wherein oxygen reacts with said photoluminescent molecules to alter characteristics of said light emitted from said photoluminescent molecules, wherein said at least one photo-detector is adapted to detect said light emitted from said photoluminescent molecules and to provide output signals representative of said characteristics to determine said concentration of oxygen.

4. Medical device according to claim 1, wherein the said anchor means comprises at least two hatches separately arranged along said distal tip of said electrode lead.

5. Medical device according to claim 1, wherein said control means is adapted to generate said indication signal if said measurement signal is below said oxygen level threshold longer than a preset time period, indicating potential interstitial edema.

6. Medical device according to claim 1, wherein said predetermined oxygen level threshold is 97 % SaO₂.

7. Medical device according to claim 1, wherein said control means is adapted to generate a second indication signal if said measuremnt signal is below a second oxygen threshold, being 92 % SaO₂ and indicating potential pulmonary edema.

8. Medical device according to claim 1, wherein said control means is configured to generate an indication signal indicative of when a patient no longer may saturate said blood during exercise.

9. Medical device according to claim 1, further comprising a heart stimulating means connected to and controlled by said control means to provide a stimulation rate that is controlled by said oxygen measurement signal.

10. Medical device according to claim 1, further comprising a pressure sensor arranged in connection with said oxygen sensor at, or close to, said distal end of said electrode lead.

11. Medical device according to claim 1, further comprising an auxiliary sensor connected to said control means and adapted to generate an auxiliary measurement signal in dependence of a measurement influencing activity, wherein said said optical oxygen sensor is controlled to perform said blood oxygen measurements when the auxiliary measurement signal fulfils specified measurement criteria.

12. Medical device according to claim 11, wherein said auxiliary sensor includes an activity sensor.

13. Medical device according to claim 11, wherein said auxiliary sensor includes a patient position sensor.

14. Medical device according to claim 1, wherein
said optical oxygen sensor is adapted to generate a pO₂ signal indicative of obtain a partial pressure of oxygen, pO₂, dissolved in said blood entering said left atrium,
said implantable medical device is adapted to calculate a SaO₂ signal corresponding to said pO₂ signal, and
said control means is adapted to compare said SaO₂ signal to a predetermined threshold level and generate, in dependence of said comparison, an indication signal indicative of lung functionality of said patient.

## Patentansprüche

1. Implantierbares Medizinprodukt, umfassend:
eine Schnittstelleneinrichtung, die mit einem Elektrodendraht verbunden ist, umfassend einen optischen Sauerstoffsensor, der dafür geeignet ist, einen Sauerstoffpegel in mit Sauerstoff versetztem Blut zu messen und ein Sauerstoffmesssignal in Abhängigkeit von dem Sauerstoffpegel zu erzeugen, wobei das implantierbare Medizinprodukt ferner ein Steuermittel umfasst, das mit der Schnittstelleneinrichtung verbunden ist und dafür geeignet ist, das Sauerstoffmesssignal mit einem vorbestimmten Schwellenpegel zu vergleichen, und in Abhängigkeit von dem Vergleich ein Anzeigesignal zu erzeugen, das anzeigend für eine Lungenfunktionalität des Patienten ist,
wobei der Sauerstoffsensor an einem distalen Ende des Elektrodendrahts angeordnet ist,
**dadurch gekennzeichnet, dass**:
der Elektrodendraht ein rechter Herzvorhofdraht ist, umfassend ein Ankermittel, um die distale Spitze des Elektrodendrahts innerhalb einer Septumwand zwischen einem rechten Vorhof und einem linken Vorhof eines Patientenherzens anzubringen, und der Sensor an der distalen Spitze angeordnet ist und der distalen Richtung des Elektrodendrahts gegenübersteht, dass ermöglicht wird, dass der Sauerstoffsensor Messungen innerhalb des Herzens an Blut durchführt, das in den linken Vorhof eintritt.

2. Medizinprodukt nach Anspruch 1, wobei der optische Sensor eine Photodiode und eine lichtemittierende Diode umfasst, die an einer distalen Spitze des Elektrodendrahts angeordnet sind und einer distalen Richtung des Elektrodendrahts gegenüberstehen.

3. Medizinprodukt nach Anspruch 1, wobei der Sauerstoffsensor umfasst:
zumindest eine Lichtquelle, die dafür geeignet ist, während Messabläufen zumindest eine erste Wellenlänge zu emittieren;
zumindest einen Photodetektor; und
zumindest einen Typ von Photolumineszenzmolekülen, die in einem Träger eingebettet sind, der gegenüber Sauerstoff selektiv permeabel ist, aufweisend ein Teil, das mit arteriellem Blut des Patienten in Kontakt ist und optisch mit der zumindest einen Lichtquelle und dem zumindest einen Photodetektor verbunden ist, wobei die Photolumineszenzmoleküle in Reaktion auf eine Anregung durch das Licht, das von der zumindest einen Lichtquelle emittiert wird, Licht emittieren, und wobei Sauerstoff mit den photolumineszierenden Molekülen reagiert, um Eigenschaften des Lichts zu ändern, das von den photolumeszierenden Molekülen emittiert wird, wobei der zumindest eine Photodetektor dafür geeignet ist, das Licht zu erfassen, das von den photolumineszierenden Molekülen emittiert wird, und Ausgangssignale bereitzustellen, die für die Eigenschaften repräsentativ sind, um die Konzentration von Sauerstoff zu bestimmen.

4. Medizinprodukt nach Anspruch 1, wobei das Ankermittel zumindest zwei Luken umfasst, die getrennt entlang der distalen Spitze des Elektrodendrahts angeordnet sind.

5. Medizinprodukt nach Anspruch 1, wobei das Steuermittel dafür geeignet ist, das Anzeigesignal zu erzeugen, wenn das Messsignal länger als eine vorbestimmte Zeitperiode unterhalb der Sauerstoffpegelschwelle liegt, was ein potentielles interstitielles Ödem anzeigt.

6. Medizinprodukt nach Anspruch 1, wobei die vorbestimmte Sauerstoffpegelschwelle 97% SaO₂ beträgt.

7. Medizinprodukt nach Anspruch 1, wobei das Steuermittel dafür geeignet ist, ein zweites Anzeigesignal zu erzeugen, wenn das Messsignal unterhalb einer zweiten Sauerstoffschwelle liegt, die 92% SaO₂ beträgt und ein potenzielles Lungenödem anzeigt.

8. Medizinprodukt nach Anspruch 1, wobei das Steuermittel dafür konfiguriert ist, ein Anzeigesignal zu erzeugen, das anzeigend dafür ist, wann ein Patient das Blut während körperlicher Anstrengung nicht mehr sättigen kann.

9. Medizinprodukt nach Anspruch 1, ferner umfassend eine Herzstimulationseinrichtung, die mit dem Steuermittel verbunden ist und von dieser gesteuert wird, um eine Stimulationsrate bereitzustellen, die durch das Sauerstoffmesssignal gesteuert wird.

10. Medizinprodukt nach Anspruch 1, ferner umfassend einen Drucksensor, der in Verbindung mit dem Sauerstoffsensor an oder nahe bei dem distalen Ende des Elektrodendrahts angeordnet ist.

11. Medizinprodukt nach Anspruch 1, ferner umfassend einen Zusatzsensor, der mit dem Steuermittel verbunden ist und dafür geeignet ist, in Abhängigkeit von einer die Messung beeinflussenden Aktivität ein Zusatzmesssignal zu erzeugen, wobei der optische Sauerstoffsensor gesteuert wird, die Blutsauerstoffmessungen durchzuführen, wenn das Zusatzmesssignal spezifizierte Messkriterien erfüllt.

12. Medizinprodukt nach Anspruch 11, wobei der Zusatzsensor einen Aktivitätssensor umfasst.

13. Medizinprodukt nach Anspruch 11, wobei der Zusatzsensor einen Patientenpositionssensor umfasst.

14. Medizinprodukt nach Anspruch 1, wobei
der optische Sauerstoffsensor dafür geeignet ist, ein pO₂-Signal zu erzeugen, das anzeigend dafür ist, dass ein Partialdruck von Sauerstoff pO₂ erhalten wird, der in dem Blut gelöst ist, das in den linken Vorhof eintritt,
das implantierbare Medizinprodukt dafür geeignet ist, ein SaO₂-Signal zu berechnen, das dem pO₂-Signal entspricht, und
das Steuermittel dafür geeignet ist, das SaO₂-Signal mit einem vorbestimmten Schwellenpegel zu vergleichen und in Abhängigkeit von dem Vergleich ein Anzeigesignal zu erzeugen, das anzeigend für eine Lungenfunktionalität des Patienten ist.

## Revendications

1. Dispositif médical implantable comprenant :
un moyen d'interface relié à un fil d'électrode comprenant un capteur d'oxygène optique adapté pour mesurer un taux d'oxygène dans du sang oxygéné et pour générer un signal de mesure d'oxygène en fonction dudit taux d'oxygène, ledit dispositif médical implantable comprenant en outre un moyen de commande relié audit moyen d'interface et adapté pour comparer ledit signal de mesure d'oxygène à un niveau seuil prédéterminé et pour générer, en fonction de ladite comparaison, un signal indicateur de la fonctionnalité des poumons dudit patient,
ledit capteur d'oxygène étant agencé à une extrémité distale dudit fil d'électrode,
**caractérisé en ce que** :
ledit fil d'électrode est un fil auriculaire droit comprenant un moyen d'ancrage destiné à fixer la pointe distale dudit fil d'électrode à l'intérieur de la paroi septale entre l'oreillette droite et l'oreillette gauche du coeur d'un patient, et **en ce que** ledit capteur est agencé sur la pointe distale en faisant face au sens distal dudit fil d'électrode, de manière à permettre audit capteur d'oxygène d'effectuer des mesures, à l'intérieur dudit coeur, relatives au sang pénétrant dans ladite oreillette gauche.

2. Dispositif médical selon la revendication 1, dans lequel ledit capteur optique comprend une photodiode et une diode électroluminescente agencées sur une pointe distale dudit fil d'électrode et orientées dans le sens distal dudit fil d'électrode.

3. Dispositif médical selon la revendication 1, dans lequel ledit capteur d'oxygène comprend :
au moins une source lumineuse adaptée pour, au cours de sessions de mesure, émettre de la lumière à au moins une première longueur d'onde ;
au moins un photodétecteur ; et
au moins un type de molécules photoluminescentes incorporées dans un support sélectivement perméable à l'oxygène, présentant une partie qui est en contact avec le sang artériel dudit patient et reliées optiquement à ladite au moins une source lumineuse et audit au moins un photodétecteur, lesdites molécules photoluminescentes émettant de la lumière en réaction à l'excitation résultant de ladite lumière émise par ladite au moins une source lumineuse, l'oxygène réagissant avec lesdites molécules photoluminescentes pour modifier les caractéristiques de ladite lumière émise par lesdites molécules photoluminescentes, ledit au moins un photodétecteur étant adapté pour détecter ladite lumière émise par lesdites molécules photoluminescentes et pour produire des signaux de sortie représentatifs desdites caractéristiques pour déterminer ladite concentration d'oxygène.

4. Dispositif médical selon la revendication 1, dans lequel ledit moyen d'ancrage comprend au moins deux trappes agencées séparément le long de ladite pointe distale dudit fil d'électrode.

5. Dispositif médical selon la revendication 1, dans lequel ledit moyen de commande est adapté pour générer ledit signal indicateur si ledit signal de mesure demeure inférieur audit niveau seuil d'oxygène plus longtemps qu'une période de temps préétablie, indiquant un oedème interstitiel potentiel.

6. Dispositif médical selon la revendication 1, dans lequel ledit niveau seuil d'oxygène prédéterminé correspond à une SaO₂ de 97 %.

7. Dispositif médical selon la revendication 1, dans lequel ledit moyen de commande est adapté pour générer un second signal indicateur si ledit signal de mesure est inférieur à un second seuil d'oxygène, correspondant à une SaO₂ de 92 % et indiquant un oedème pulmonaire potentiel.

8. Dispositif médical selon la revendication 1, dans lequel ledit moyen de commande est configuré pour générer un signal indicateur du moment où un patient ne peut plus saturer son sang au cours de périodes d'exercice.

9. Dispositif médical selon la revendication 1, comprenant en outre un moyen de stimulation cardiaque relié audit moyen de commande et commandé par ce dernier pour fournir un rythme de stimulation qui est commandé par ledit signal de mesure d'oxygène.

10. Dispositif médical selon la revendication 1, comprenant en outre un capteur de pression agencé en liaison avec ledit capteur d'oxygène sur l'extrémité distale dudit fil d'électrode ou à proximité de celle-ci.

11. Dispositif médical selon la revendication 1, comprenant en outre un capteur auxiliaire relié audit moyen de commande et adapté pour générer un signal de mesure auxiliaire en fonction d'une activité influant sur la mesure, ledit capteur d'oxygène optique étant commandé pour effectuer lesdites mesures de l'oxygène sanguin lorsque le signal de mesure auxiliaire remplit des critères de mesure spécifiques.

12. Dispositif médical selon la revendication 11, dans lequel ledit capteur auxiliaire comprend un capteur d'activité.

13. Dispositif médical selon la revendication 11, dans lequel ledit capteur auxiliaire comprend un capteur de position du patient.

14. Dispositif médical selon la revendication 1, dans lequel
ledit capteur d'oxygène optique est adapté pour générer un signal pO₂ indicateur de l'obtention d'une pression partielle d'oxygène, pO₂, dissous dans ledit sang pénétrant dans ladite oreillette gauche,
ledit dispositif médical implantable est adapté pour calculer un signal SaO₂ correspondant audit signal pO₂, et
ledit moyen de commande est adapté pour comparer ledit signal SaO₂ à un niveau seuil prédéterminé et pour générer, en fonction de ladite comparaison, un signal indicateur de la fonctionnalité des poumons dudit patient.
